# EUROPEAN PATENT APPLICATION

(11) **EP 2 138 588 A1**
(43) Date of publication of application: **30.12.2009**
(21) Application number: 08158784.2
(22) Date of filing: 23.06.2008
(51) Int. Cl.: C12Q 1/68

(54) **Melting curve measurement during amplification**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to a process for conducting melting curve measurement during amplification. The invention is especially suited for conducting melting curve measurement during simultaneous identification of multiple nucleic acids present in a sample, e.g. a biological sample.

The methods of the present invention may be used for clinical diagnostics, for point-of-care diagnostics, for bio-molecular diagnostics, for gene or protein expression arrays, for environmental sensors, and for food quality sensors.

## Description

### FIELD OF THE INVENTION

The present invention relates to a process for conducting melting curve measurement.
The methods of the present invention may be used for clinical diagnostics, for point-of-care diagnostics, for bio-molecular diagnostics, for gene or protein expression arrays, for environmental sensors, and for food quality sensors.

### BACKGROUND OF THE INVENTION

Polymerase chain reaction (PCR) is a method for amplification of specific nucleic acid sequences. PCR is an enzymatic reaction that takes place in solution or with surface immobilized primers. When the amplification process is monitored in real-time, PCR can be used for quantitative analysis. Real-time PCR normally uses fluorescent reporters, such as intercalating dyes, TaqMan® probes, Scorpion primers, molecular beacons.

Methods for quantifying the specificity of the binding of the amplified nucleic acid sequences to the capture probes are known in the art. This specificity is analyzed in order to discriminate between specific and aspecific binding of the amplified nucleic acid sequences to capture probes. Normally, a melting curve analysis as a single measurement is done after having performed the PCR reaction or after hybridization of the PCR products to capture probes in order to discriminate between specific and aspecific binding.

EP-B1 0 906 449 describes systems and methods for carrying out and monitoring polymerase chain reaction wherein continuous monitoring of probe fluorescence within a PCR cycle is conducted for assessing PCR products and probe melting curves to identify amplification products and mutations.

### SUMMARY OF THE INVENTION

An object of the invention is to provide an improved method of detecting the specificity of the binding of the amplified nucleic acid sequences to the capture probes in amplification methods.

Subject of the present invention is a method for detecting a nucleic acid comprising:
providing a target nucleic acid to be detected,
a first step of amplifying the target nucleic acid,
a binding step in which amplified target nucleic acid binds to a surface-immobilized capture probe,
detecting said amplified target nucleic acid,
a step of measuring a melting curve of the amplified target nucleic acid bound to the capture probe,
and a second step of amplifying the target nucleic acid.

Measuring the melting curve may already encompass the detection of said bound amplified target nucleic acid. It should be understood that it is not strictly necessary that the detection step takes place as an extra step before the measurement of the melting curve. Measuring the melting curve and detection may take place subsequently but may also take place concurrently. Measuring the melting curve takes place between the first and second amplification step. The melting curve is measured before the second amplification step.

In a preferred embodiment of the method according to the invention the melting curve is analyzed for assessing the specificity with which the amplified target nucleic acid is bound to the capture probe.

In an especially preferred embodiment of the method according to the invention the method further comprises a step of adjusting a parameter for controlling the second and/or (a) subsequent step(s) of amplifying the target nucleic acid.

In one embodiment of the method according to the invention amplifying the target nucleic acid involves a temperature decrease and wherein the temperature decrease is used for measuring the melting curve. In another embodiment of the method according to the invention amplifying the target nucleic acid involves a temperature increase and wherein the temperature increased is used for measuring the melting curve. The latter one is more preferred.

In another embodiment of the method according to the invention the detection of said amplified nucleic acid sequences captured to said capture probes is performed with a surface-specific detection method detecting a signal in proximity to the surface.

In a preferred embodiment of the present invention the melting curves are measured and analyzed during more than one cycle of the amplification.

In another embodiment of the method according to the invention the detection of amplified target nucleic acids is a quantitative detection.

In another preferred embodiment the present invention is carried out as multiplex method wherein multiple target nucleic sequences are simultaneously amplified in a single container. In this case multiple primer pairs may be used for the amplification reaction of multiple targets.

In another preferred embodiment of the method according to the invention multiple target nucleic sequences are simultaneously and real-time detected in a single container.

Subject of the invention is further a method of analyzing a sample for the presence of a target nucleic acid comprising the method of detecting said target nucleic acid to be detected. This method comprises the steps of:
providing a sample to be analyzed for the presence of said target nucleic acid,
a first step of amplifying the target nucleic acid if present in said sample,
a binding step in which amplified target nucleic acid binds to a surface-immobilized capture probe,
detecting for the presence of amplified target nucleic acid,
a step of measuring a melting curve of the amplified target nucleic acid bound to the capture probe,
and a second step of amplifying the target nucleic acid if present in said sample.

Subject of the present invention is also a device for performing a method according to the present invention comprising:
a reaction compartment for the amplification of a target nucleic acid,
a hybridization surface comprising a surface-immobilized capture probe, temperature varying and temperature control means,
a melting curve measurement system,
a detection system for detecting target nucleic acids captured to the surface-immobilized capture probes.

Subject of the present invention is also a device for performing a method according to the present invention comprising:
a reaction compartment for the amplification of a target nucleic acid,
means for performing an amplification step,
a hybridization surface comprising a surface-immobilized capture probe,
a detection system for detecting target nucleic acids captured to the surface-immobilized capture probes,
a melting curve measurement system comprising:
   temperature varying and temperature control means,
   a detection system for detecting target nucleic acids captured to the surface-immobilized capture probes.

The methods and devices of the present invention may be used for clinical diagnostics, for point-of-care diagnostics, for bio-molecular diagnostics, for gene or protein expression arrays, for environmental sensors, and for food quality sensors.

### BRIEF DESCRIPTION OF THE DRAWINGS

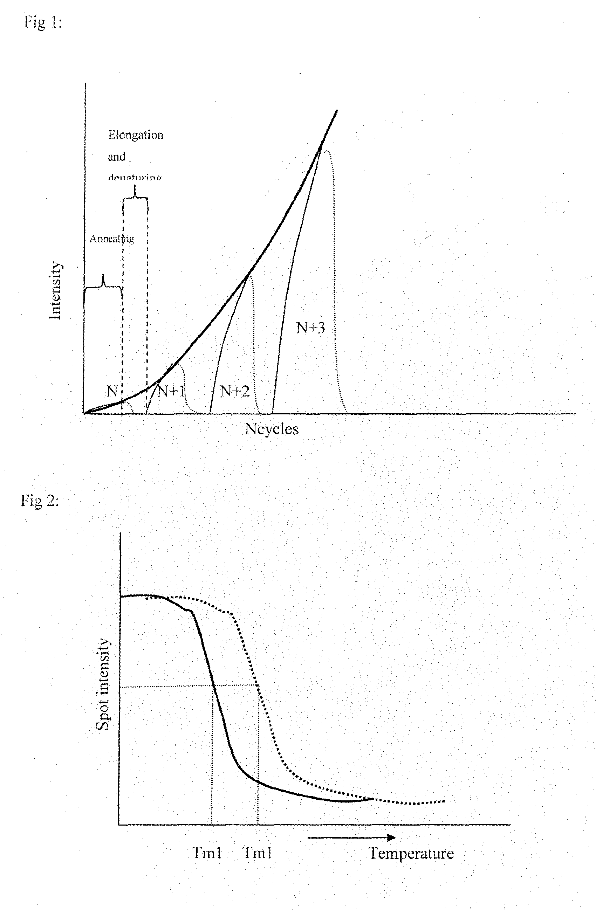

Fig. 1: The theoretical intensity of surface hybridized amplicons is displayed as a function of the number of cycles. The intensity decreases to zero during the denaturing step of the PCR. During the next PCR cycle, the intensity increases to a higher level compared to the previous cycle as target DNA has been multiplied during the previous cycle. The fat black line depicts the overall hybridization signal.
Fig. 2: The intensity of the spot of an aspecifically bound molecule (fat black) and an exactly complimentary molecule (dotted). The melting temperature Tm is defined as the temperature at which 50% of the molecules are still bound to the complimentary part. The melting temperature of the aspecifically bound molecule is lower than the perfect match.

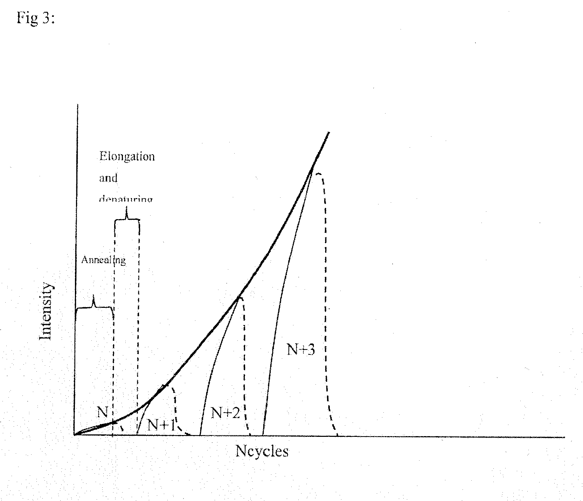

Fig. 3: Spot intensity of surface immobilized amplicons as a function of the number of cycles. The dotted lines denote the melting curves, the fat black line the overall hybridization intensities.

### DETAILED DESCRIPTION OF EMBODIMENTS

Subject of the present invention is a method for detecting a nucleic acid comprising:
providing a target nucleic acid to be detected,
a first step of amplifying the target nucleic acid,
a binding step in which amplified target nucleic acid binds to a surface-immobilized capture probe,
detecting said amplified target nucleic acid,
a step of measuring a melting curve of the amplified target nucleic acid bound to
the capture probe,
and a second step of amplifying the target nucleic acid.

Measuring the melting curve may already encompass the detection of said bound amplified target nucleic acid. It should be understood that it is not strictly necessary that the detection step takes place as an extra step before the measurement of the melting curve. Measuring the melting curve and detection may take place subsequently but may also take place concurrently. Measuring the melting curve takes place between the first and second amplification step.

Instead of providing a target nucleic acid a sample may be provided that is analyzed for the presence of such target nucleic acid. During the amplification steps an amplification mixture is provided and amplification takes place in case the target nucleic acid is present in the provided sample. An amplification mixture is known in the art and commercially available. It may comprise an amplification enzyme, preferably a thermostable enzyme, it may comprise primer or a primer pair. The sample which may be provided may be a biological sample, blood, urine, saliva, CSF, sputum, BAL, Tracheal aspirate, wound swap, tissue or stool.

Thus, subject of the invention is a method of analyzing a sample for the presence of a target nucleic acid comprising the method of detecting said target nucleic acid to be detected. This method comprises the steps of:
providing a sample to be analyzed for the presence of said target nucleic acid,
a first step of amplifying the target nucleic acid if present in said sample,
a binding step in which amplified target nucleic acid binds to a surface-immobilized capture probe,
detecting for the presence of amplified target nucleic acid,
a step of measuring a melting curve of the amplified target nucleic acid bound to
the capture probe,
and a second step of amplifying the target nucleic acid if present in said sample.

It is to be understood that the method for detecting a nucleic acid may encompass preceding and subsequent amplification steps. The above method may be part of a method comprising a number of amplification cycles.

In a preferred embodiment of the method according to the invention the melting curve is analyzed for assessing the specificity with which the amplified target nucleic acid is bound to the capture probe.

That means in an embodiment of the invention the melting curves are analyzed in order to discriminate between specific and aspecific binding of the amplified nucleic acid sequences to the capture probes and in case of need the amplification parameters may be adjusted in order to improve the specificity of binding. This adjustment may comprise an annealing temperature increase.

Thus, in an especially preferred embodiment of the method according to the invention the method further comprises a step of adjusting a parameter for controlling subsequent amplification cycles. It may be an adjustment of the second and/or (a) subsequent step(s) of amplifying the target nucleic acid; that means adjusting the parameters of the amplification step. It may be also a preferred embodiment to adjust a parameter of a binding step, e.g. a change in the hybridization temperature.

Increasing the annealing temperature of the amplification step may improve the performance if the amplification results in too much aspecific product which is detected on the capture probes.

Increasing the hybridization temperature may improve the process if aspecific binding of the target to the probe itself (too less stringent conditions) causes the problem. Or a combination of both may be advantageously should the situation arise.

In case an adjustment of parameters is conducted it may also be preferred to repeat the binding step with adjusted parameters before performing the next amplification step. It is an alternative in case an adjustment of parameters is conducted that the next amplification step is performed instead of repeating the binding step.

In one embodiment of the method according to the invention amplifying the target nucleic acid involves a temperature decrease and the temperature decrease is used for measuring the melting curve. In another embodiment of the method according to the invention amplifying the target nucleic acid involves a temperature increase and the temperature increase is used for measuring the melting curve. The latter embodiment is more preferred as the temperature increase can be coincide with the temperature increase needed to go from elongation to denaturation temperature, eliminating the need for an additional temperature step.

In another embodiment of the method according to the invention the detection of said amplified nucleic acid sequences captured to said capture probes is performed with a surface-specific detection method detecting a signal in proximity to the surface. This has the advantage of a reduced background signal, which makes the method faster or more sensitive.

In a preferred embodiment of the present invention the melting curves are measured and eventually analyzed during more than one cycle of the amplification, preferably during more than two or three cycles, more preferably during every second cycle or if desired during every cycle.

More preferred embodiment is to measure and analyze it only after cycle N, whereby N is determined by the cycle during which the signal passes threshold. This will give more valuable and robust information than a signal measurement. Furthermore, the result of a parameter adjustment during amplification (e.g. temperature increase during annealing to form more specific products) can be measured during a following melting curve analysis and compared with previous measurements.

In another embodiment of the method according to the invention the detection of amplified target nucleic acids is a quantitative detection to be able to detect the number of targets originally present in the sample.

More preferred, the method according to the present invention is conducted in a single container or in a single compartment. This makes the cartridge simple and cheap. Furthermore, it reduces chances of cross-contamination as the sample does not have to be manually transferred to different containers.

In another preferred embodiment of the method according to the invention multiple target nucleic sequences are simultaneously and real-time detected in a single container.

In another preferred embodiment of the method according to the invention amplifying the target nucleic acid involves PCR. Instead of PCR one can of course also use other amplification methods such as, but not limited to, NASBA, TMA, RCA.

In another preferred embodiment of the method according to the invention the amplification takes place in a container comprising a microarray.

It is preferred according to the present invention that the detected signal is a fluorescence signal.

Another embodiment of the invention is a method for detection of target nucleic acid sequences during amplification,
wherein detection occurs during the amplification cycle, and wherein detection of said amplified nucleic acid sequences is performed when said amplified nucleic acid sequences are bound to surface-immobilized capture probes during the amplification cycle, and
wherein the melting curve of said amplified nucleic acid sequences bound to the capture probes is concurrently measured at least once during the performance of the method.

In a preferred embodiment of the above method it is a method for real-time amplification and detection. More preferred as detection is a quantitative detection. All preferred embodiments of the method for amplifying a nucleic acid as described above and below are also preferred embodiments of the described method for detection of target nucleic acid sequences during amplification.

In one embodiment the method of the present invention represents a method comprising a combined amplification and hybridization step wherein melting curves are analyzed during said real-time amplification detection. This reduces the overall process time.

In a preferred embodiment of the method the melting curves are measured for the first time after the cycle where hybridization signal is detected. In another preferred a melting curve is measured after every Xth cycle, wherein X is 2, 3, 4, 5, 6, 7, 8, 9 or 10.

In a another method for real-time quantitative detection of target nucleic acid sequences during amplification according to the present invention the melting curves are measured and analyzed during every cycle of the amplification.

In another preferred embodiment of the invention multiple target nucleic sequences are simultaneously and real-time quantitatively detected and multiple melting curves are measured and analyzed.

It may be preferred that the PCR mixture can be enriched for single stranded amplicons by means of asymmetrical PCR or LATE PCR (LATE: linear after the exponential). In asymmetrical PCR, unequal concentrations of forward and reverse PCR primer are used. When the concentration of the labeled primer is higher than of the unlabeled primer, the labeled strand will be amplified at a higher rate than the unlabeled strand. This not only leads to an accumulation of the labeled strand but also directly favors the hybridization of the labeled strand to the capture probe. LATE PCR is described in Sanchez et al.; PNAS (2004) Vol. 101, no.7, pages 1933-1938 and in Pierce et al. PNAS, (2005), Vol. 102, no.24, pages 8609-8614.

In one embodiment of the present invention the detected signal intensity correlates to the number of amplified nucleic acid sequences bound to the capture probes and the characteristics of the curve and also the melting curve correlate to the specificity of that binding in the method according to the present invention, which may be preferably a method for real-time quantitative detection of target nucleic acid sequences during amplification.

The advantage of a combined amplification (as e.g. PCR) and hybridization step as used in an embodiment of the method of the present invention is a short time to obtaining results combined with a higher possible multiplex grade.

The amplification step of the method according to the present invention may be conducted isothermically and non-isothermically. In case the method is a non-isothermic amplification reaction, e.g. PCR, a temperature increase occurs during every cycle of the amplification reaction, and this potentially provides a melting curve during every cycle.

One essential feature of one embodiment of the methods according to the present invention is that information obtained from the intensity plots during potentially every cycle can be used for addressing the specificity without any additional process time and even to adapt the temperature profile to correct for a sub-optimal amplification process (e.g., lack of stringency). Because every cycle of a non-isothermic amplification reaction contains a temperature ramp-up, the spot intensities during these temperature ramp-ups may be used to qualify the specificity of the assay during every amplification cycle.

For illustration of the invention we will describe an embodiment of the invention in the following. It should be understood that this is by no means meant to be limiting.

A PCR chamber comprises a microarray with different capture probe spots printed. Each spot contains molecules (i.e., capture probes) that are exactly complementary to [a fraction of the sequence of] a specific target molecule. During the annealing step of the PCR where the primers hybridise to the targets, the temperature is such that the targets can also hybridise to the capture probes on the microarray. A number of target molecules will thus not be amplified during one cycle but will hybridize onto the capture probes, which can be detected by a surface specific technique.

During the denaturation step, the target-probe complexes denature again and the target is again in solution. In figure 1, the theoretical fluorescent intensity of a spot is depicted against the number of temperature cycles. The part of the graph is in the exponential phase of the PCR, during every cycle the number of targets is multiplied (theoretically with a maximal factor of 2) and thus the fluorescent intensity measured during the annealing step also increases with every cycle. In between two cycles, the intensity returns to zero as the high temperature of the denaturation step results in de-binding of the targets to the probes.

A PCR cycle contains two temperature ramp-up moments, namely from annealing to elongation, and from elongation to denaturation. During these temperature ramp-ups, the intensity of the spots is detected as a function of temperature. After every cycle, by comparing the expected melting temperature with the resulted melting temperature, it can be concluded to what extent aspecific binding plays a role and one can correct the detected concentration of hybridized PCR strands for unspecific binding. In figure 3 the dotted lines are the melting curves that need to be analyzed.

Aspecifically bound molecules (where there is a least one base-pair mismatch between the sequence of the capture probe and the bound fragment of the sequence bound molecule) have a lower melting temperature, see figure 2, and thus the bonds with the capture probe will break at a lower temperature during the denaturation than an exactly complementary molecule. By measuring the melting temperatures and comparing these to the expected values, specificity can be assessed.

According to the prior art methods this takes additional process time. Furthermore, the methods of the prior art are not very robust, as only at one moment during the assay, information is obtained on specificity. One error (air bubbles, array out of focus, particles on the array) may completely render the melting curve analysis useless.

Furthermore, in case the assay has been done under too less stringent conditions (e.g. too low temperature during the annealing / hybridization process step), this will only be concluded at the end of the assay according to the methods of the prior art. There is no possibility for adjusting the temperatures of annealing in the prior art methods.

Thus the advantages of the method according to the present invention are the following:

There is more information available during an early stage of the process. This makes the melting curve analysis much more robust in comparison to the prior art methods.

Because of the availability of this information in an early stage during the amplification, the amplification parameters can be adjusted to improve specificity (e.g. annealing temperature increase).

No additional process time may be required for determining the specificity of binding in case the temperature step is part of the amplification performed.

Compared to the standard way of performing a melting curve analysis after the hybridization, this analysis is much more robust because much more information is available and often small differences in melting temperatures need to be determined. The total number of melting curves available for specificity analysis depends on the number of cycles and the Ct-value (i.e., the number of cycles needed to have a signal above the detection limit).

Furthermore, the early stage during which information is available on specificity gives the opportunity to adjust specific assay parameter real-time. E.g. in case too much aspecific product is formed during PCR, the temperature annealing step can be increased in order to obtain more specific PCR product and more specific hybridization. This increases the quality and yield of the performed assays.

In the following a non-limiting embodiment using LATE PCR is described below:

As an example using LATE PCR, the sample, and PCR mix (consisting of a PCR buffer with enzyme, nucleotides and salts) and a labeled and unlabelled primer are introduced in a single container. The labeled and unlabeled primers are in the concentration ratio of 10: 1 respectively and the melting temperature of the labeled primer is 5 °C lower than the melting temperature of the unlabelled primer.

Amplification and hybridization are performed in a single chamber and hybridization takes place at the hybridization temperature which is set on the average melting temperature of the two primers.

Subject of the present invention is also a device for performing a method according to the present invention comprising:
a reaction compartment for the amplification of a target nucleic acid,
a hybridization surface comprising a surface-immobilized capture probe,
temperature varying and temperature control means,
a melting curve measurement system,
a detection system for detecting target nucleic acids captured to the surface-immobilized capture probes.

Subject of the present invention is also a device for performing a method according to the present invention comprising:
a reaction compartment for the amplification of a target nucleic acid,
a hybridization surface comprising a surface-immobilized capture probe,
a detection system for detecting target nucleic acids captured to the surface-immobilized capture probes,
a melting curve measurement system comprising:
   temperature varying and temperature control means,
   a detection system for detecting target nucleic acids captured to the surface-immobilized capture probes.

In a preferred embodiment of the device the device further comprises means for performing an amplification step. In non-isothermic amplification method this may be temperature varying and temperature control means.

It is to be understood that detection system for detecting a target nucleic acid captured to the surface-immobilized capture probe may be used for detection as well as for measurement of the melting curve. The detection system may encompass in case of fluorescence measurement a source for excitation of fluorescence and a detector measuring the emitted fluorescence. The device may have the same detection system for detection as well as for measurement of the melting curve, but it may have several and/or different detection systems. The same or different temperature varying and temperature control means may be used for performing an amplification step and for melting curve measurement.

It is required that one must be able to accurately and rapidly change the temperature during the melting curve measurement and measure the spot intensities fast enough.

In a preferred embodiment the devices comprises an algorithm that causes, when executed, said device to carry out the method steps according to the invention.

In a preferred embodiment of the device according to the present invention the device comprises means for performing an algorithm that carries out the method steps according to the method of the present invention.

Subject of the present invention is also the use of a method according to the present invention and the use of a device according to the present invention for clinical diagnostics, for point-of care diagnostics, for bio-molecular diagnostics, for gene or protein expression arrays, for environmental sensors, for food quality sensors.

A further subject of the present invention is the use of a method according to the present invention and the use of a device according to the present invention for analyzing a sample for the presence of a target nucleic acid.

According to the present invention the amplification method may be selected from the group comprising polymerase chain reaction (PCR), NASBA (nucleic acid sequence based amplification), TMA (transcription mediated amplification), and rolling circle amplification. Enzymatic amplification methods suitable for the present invention are known to a person skilled in the art.

In a preferred embodiment of the invention the amplification reaction is PCR.

A PCR reaction may be conducted as follows:

Template DNA together with a Mastermix (containing the thermostable DNA polymerase, nucleotides, magnesium salts and additional salts, stabilizers etc.), and forward and reverse primers (one of the primers may be labeled e.g. labeled with a fluorophore) are all added into a volume. This volume is subjected to temperature cycling, in order to amplify the amount of target DNA.

A three-step PCR cycle may consist of the following temperature steps:
Denaturation step: This step is the first regular cycling event and consists of heating the reaction to 90-98°C for 20-30 seconds. It causes melting of DNA template and primers.
Annealing step: The reaction temperature is lowered to 45-65°C for 20-40 seconds allowing annealing of the primers to the single-stranded DNA template.
Extension step: The temperature at this step depends on the DNA polymerase used; Taq polymerase has its optimum activity temperature at 75-80°C, and commonly a temperature of 72°C is used with this enzyme. At this step the DNA polymerase synthesizes a new DNA strand complementary to the DNA template strand by adding dNTP's that are complementary to the template in 5' to 3' direction.

A person skilled in the art is aware that the above is a non-limiting example of a PCR reaction. Times, temperatures and agents may be varied. Other polymerases are e.g. known in the art as e.g. *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerase, *Pyrococcus furiosus* (Pfu) DNA polymerase, *Pyrococcus woesei* (Pwo) DNA polymerase, *Pyrococcus kodakaraensis* KOD DNA polymerase, *Thermus filiformis* (Tfi) DNA polymerase, *Sulfolobus solfataricus* Dpo4 DNA polymerase, *Thermus pacificus* (Tpac) DNA polymerase, *Thermus eggertsonii* (Teg) DNA polymerase and *Thermus flavus* (Tfl) DNA polymerase.. PCR may be conducted with mixtures of polymerases or mutants or recombinant types of polymerases. All these variations are known to a person skilled in the art.

In a preferred embodiment of the method for real-time quantitative detection of target nucleic acid sequences during amplification according to the present invention the capture probes are surface-immobilized and the detection of said amplified nucleic acid sequences captured to said capture probes is performed with a surface-specific detection method detecting a signal in proximity to the surface.

Surface specific detection means that the contribution to the detected signal by amplicons that are not captured (e.g., floating in the fluid on top of the surface with capture probes) is substantially suppressed, which means preferably suppressed by at least a factor 50, more preferably by at least a factor 100, and even more preferably by at least a factor 1000, while the contribution to the detected signal by captured amplicon is (substantially) not suppressed.

As an example the case of a fluid cell having a height of 500 microns and containing labeled primers at a concentration of 1 micromolar is considered:

Without surface specific detection, the labeled primers give rise to a detected signal equivalent to ~300000 labels per square micrometer. For a capture probe density of 10000 capture probes per square micrometer, in the best case this would result in a signal over background ratio of 1/30. For a typical hybridization experiment not all capture probes have bound to a labeled amplicon and the signal over background ratio will be even smaller; e.g., 1/300 for 1000 captured amplicons per square micrometer.

With surface specific detection, the labeled primers in solution give rise to a substantially lower detected signal; e.g., detected signal equivalent to -300 labeled primers per square micrometer for a background suppression factor of 1000. With surface-specific detection, the signal over background ratio is substantially improved to about 3 for 1000 captured labeled amplicons per square micrometer.

Another way to describe surface specific detection is to describe a surface-specific detector that detects those labels which are bound to the surface but does essentially not detect labels which are in solution. This means that the above definition given for surface specific detection applies equally to the term "a surface-specific detector that detects those labels which are bound to the surface but does essentially not detect labels which are in solution".

The surface-specific detection system is preferentially selected from a group comprising a confocal measurement device, a plasmonic measurement device and a device for the measurement according to evanescent detection.

As mentioned above, to be able to discriminate between hybridized amplicons and primers or amplicons in solution, it is preferred to make a surface specific measurement. A surface specific measurement only detects labels that are very close to the capture surface. Because hybridization can only take place where capture probes have been deposited and the PCR mixture is homogeneous, it is possible to subtract the background (the fluorescence intensity between the spots) and to determine the amount of hybridized amplicons.

Possible labels are fluorescent labels or non-fluorescent (e.g. particulate) where a difference in refractive index or absorption can be detected by optical means. It should be straightforward for someone skilled in the art to know suitable fluorescent or non-fluorescent labels.

For highly surface specific measurements, that is a suppression of the background by at least a factor 50, one can distinguish between three different approaches:
1. Confocal: typical measurement height along the optical axis of 1-2 µm.
2. Plasmonic: measurement height of about the wavelength of excitation light or smaller.
3. Evanescent: measurement height of 100 nm or smaller.

### 1. Confocal

Confocal measurements can be made with various types of imaging equipment. Such systems can be made very compact (PCT/IB2007/052499, PCT/IB2007/052634, and PCT/IB2007/052800). Different locations along the optical axis of the system give rise to different locations where the labels are imaged by the objective closest to the array surface. By using a pinhole and proper positioning -at the location where there is a sharp image of a label at the array surface- one can select a small measurement volume (depth along the optical axis of only 1-2 µm) in the neighborhood of the sensor surface.

### 2. Plasmonic

Here the substrate is covered with a metal such as Au, Ag. The capture probes are on the metal layer or a spacing layer is deposited on top of the metal and subsequently covered with capture probes. The fluorescence of the labels of the hybridized DNA can couple to the surface plasmon at the metal medium/fluid interface. Labels in the fluid cannot couple or with a substantially smaller efficiency to the surface plasmon. By out coupling of the surface plasmon (that is converting the surface plasmon mode in to a propagating wave) and measuring the out coupled power, one essentially only measures the fluorescence of the labels of the hybridized DNA. As a result the fluorescence measurement is highly surface specific.

### 3. Evanescent

As another alternative method for enhancing the surface specificity, one can use evanescent excitation methods, where an evanescent wave is excited at the surface of the substrate and excites the fluorophores.

As a first method one can use total internal reflection (TIR) at the substrate-fluid interface, which results in measurement (excitation) volumes typically within 100-200 nm of the array surface. TIR however requires the use of a glass prism connected to the substrate or the use of a substrate with a wedge shape to enable the coupling of excitation light with angles above the critical angle of the substrate-fluid interface into the substrate.

Alternatively (as described in PCT/IB2006/051942, PCT/IB2006/054940) one can cover the substrate with a non-transparent medium such as a metal and pattern the metal with [an array of] apertures with at least one dimension in the plane parallel to the substrate-fluid interface below the diffraction limit of light in the fluid. As an example, one can pattern the substrate with wire grids that have one in-plane dimension above and the other dimension below the diffraction limit of the light in the fluid. This results in excitation volumes within 50 nm of the array surface. Advantage of this method over the first method [for evanescent excitation] is that it is simpler- there is no need for a prism or wedge shaped surface and that there are no special requirements for the angle of incidence and shape of the excitation spot and one can use a simple CCD camera for imaging the fluorescence- and enables substantially smaller excitation volumes.

In another preferred embodiment of the invention the amplification reaction is performed in a container comprising a microarray.

"Micro-array" means a support on which multiple capture molecules are immobilized in order to be able to bind to the given specific target molecule. The micro-array is preferentially composed of capture molecules present at specifically localized areas on the surface or within the support or on the substrate covering the support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and can be visualized by the detector. In one particular application of this invention, micro-arrays of capture molecules are also provided on different or separate supports as long as the different supports contain specific capture molecules and may be distinguished from each other in order to be able to quantify the specific target molecules. This can be achieved by using a mixture of beads which have particular features and are able to be distinguishable from each other in order to quantify the bound molecules. One bead or a population of beads is then considered as a spot having a capture molecule specific to one target molecules.

Micro-arrays are preferentially obtained by deposition of the capture molecules on the substrate which is done by physical means such as pin or "pin and ring" touching the surface or by release of a micro-droplet of solution by methods such as piezo- or nanodispenser.

Alternatively, in situ synthesis of capture molecules on the substrate of the embodiments of the inventions with light spatial resolution of the synthesis of oligonucleotides or polynucleotides in predefined locations such as provided by US 5,744,305 and US 6,346,413.

According to a preferred embodiment the capture probes are immobilized on the hybridization surface in a patterned array. According to a preferred embodiment the capture probes are immobilized on the surface of micro-arrays.

The capture probe molecule can be a DNA, RNA, PNA (peptide nucleic acid), LNA (locked nucleic acid), ANA (arabinonucleic acid), or HNA (hexitol nucleic acid) oligonucleotide. RNA, PNA, LNA, and HNA are able to form heteroduplexes with DNA that are more stable than DNA:DNA homoduplexes. This ensures enhanced discrimination ability for sequence mismatches (more specific hybridization). The higher stability of heteroduplexes also allows the use of shorter oligonucleotide probes at a given temperature reducing the chance of non-specific binding. PNA:DNA duplexes are formed independent of ionic strength of the hybridization buffer. This may enhance the hybridization efficiency in low salt PCR buffers.

It may be preferred according to the present invention to use capture probes with a higher melting temperature than the PCR primers and decreasing the temperature ramp down rate . This means that the capture probes will anneal to the amplicons at a temperature which is above the annealing temperature for the primers. Thus, denatured amplicons are allowed to hybridize to the capture probes, before primer elongation and subsequent elongation can take place.

Hybridization of a portion of the amplicons means that the concentration of amplicon can be directly calculated from the intensity of the signal measured due to hybridization of amplicons to the capture probes. If the relation between the measured signal and amplicon concentration is not linear, a correction algorithm or calibration curve may be applied in order to deduce the amplicon concentration.

The capture portion of the capture probe may contain from 10 to 200 nucleotides, preferably from 15 to 50 nucleotides, more preferably from 20 to 35 nucleotides specific for the amplicons produced during the amplification reaction. The capture probe can also contain additional nucleotide sequences that can act as a spacer between the capture portion and the surface or that can have a stabilizing function that can vary from 0 to 200 nucleotides, preferably from 0 to 50. These non-capturing nucleotide sequences can either contain normal nucleotides or a-basic nucleotides.

The capture molecule may be immobilized by its 5' end, or by its 3' end or random immobilization by preferred immobilization of one of the nucleotides to the functionalized support. For multiplexing, capture molecules are immobilized in specifically localized areas of a solid support in the form of a micro-array of at least 4 capture molecules per µm², preferably at least 1000 capture molecules per µm², more preferably at least 10000 capture molecules per µm², and even more preferably 100000 capture molecules per µm².

In a specific embodiment, the capture molecules comprise a capture portion of 10 to 100 nucleotides that is complementary to a specific sequence of the amplicons such that said capture potion defines two non-complementary ends of the amplicons and a spacer portion having at least 20 nucleotides and wherein the two non-complementary ends of the amplicons comprise a spacer end and a non-spacer end, respectively, such that the spacer end is non-complementary to the spacer portion of the capture molecule, and said spacer end exceeds said non-spacer end by at least 50 bases. Such spacer and capture probes are described in EP 1 788 098.

In another embodiment of the invention the capture probes on the solid surface in the PCR compartment are folded probes (e.g. molecular beacons) or other probes (e.g. TaqMan^{®} probes) with a fluorescent label and quencher in close proximity to one another due to the structure of the probe. In this embodiment the PCR reaction(s) is (are) performed without any label in the reaction or label attached to the amplification primers. Specific amplicons that are formed during the PCR reaction(s) can hybridize with the labeled capture probes on the solid surface, thereby either separating quencher and label (in case of molecular beacon type folded probes) or allowing the polymerase to hydrolyze the probe (in case of TaqMan^{®}-like capture probes). As a result a fluorescent signal can be measured at the spot where the capture probes are located.

The terms "nucleic acid, oligonucleotide, array, nucleotide sequences, target nucleic acid, bind substantially, hybridizing specifically to, background, quantifying" are the ones described in the international patent application WO 97/27317 incorporated herein by reference. The term polynucleotide refers to nucleotide or nucleotide like sequences being usually composed of DNA or RNA sequences.

The terms "nucleotides triphosphate, nucleotide, primer sequence" are further those described in the documents WO 00/72018 and WO 01/31055 incorporated herein by references.

References to nucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

The term "real-time PCR" means a method which allows detection and/or quantification of the presence of the amplicons during the PCR cycles. In real-time PCR, the presence of the amplicons is detected and/or quantified in at least one of the amplification cycles. The increase of amplicons or signal related to the amount of amplicons formed during the PCR cycles are used for the detection and/or quantification of a given nucleotide sequence in the PCR solution. In a preferred embodiment, the presence of the amplicons is detected and/or quantified in every cycle.

The term "amplicon" or amplified nucleic acid in the invention relates to the copy of the target nucleotide sequences being the product of enzymatic nucleic acid amplification.

Instead of labeled PCR primers, internal labeling with labeled dNTPs can be used.

The label-associated detection methods are numerous. A review of the different labeling molecules is given in WO 97/27317. They are obtained using either already labeled primer, or by enzymatic incorporation of labeled nucleotides during the copy or amplification step (WO 97/27329) [intercalators are not preferred in this invention].

Possible labels are fluorochromes which are detected with high sensitivity with fluorescent detector. Fluorochromes include but are not limited to cyanin dyes (Cy3, Cy5 and Cy7) suitable for analyzing an array by using commercially available array scanners (as available from, for example, General Scanning, Genetic Microsystem). FAM (carboxy fluorescein) is also a possible alternative as a label. The person skilled in the art knows suitable labels which may be used in the context of this invention.

In a preferred embodiment of the invention, a signal increase of the fluorescence signal of the array related to the presence of the amplicons on the capture molecule is detected as compared to the fluorescence in solution.

In a particular embodiment the differences of the detection of the fluorophore present on the array is based on the difference in the anisotropy of the fluorescence being associated with a bound molecule hybridized on the capture molecule as a DNA double helix compared to the fluorescence being associated with a freely moving molecule in solution. The anisotropy depends on the mobility of the fluorophores and the lifetime of the fluorescence associated with the fluorophores to be detected. The method assay for the anisotropy on array is now available from Blueshift Biotechnologies Inc., 238 East Caribbean Drive, Sunnyvale, CA 94089 (http://www.blueshiftbiotech.com/dynamicfl.html).

In a particular embodiment, the detection of fluorophore molecules is obtained preferably in a time-resolved manner. Fluorescent molecules have a fluorescent lifetime associated with the emission process. Typically lifetimes for small fluorophores such as fluorescein and rhodamine are in the 2-10 nanoscecond range. Time-resolved fluorescence (TRF) assays use a long-lived (>1000 ns) fluorophore in order to discriminate assay signal from short-lived interference such as autofluorescence of the matrix or fluorescent samples which almost always have lifetimes of much less than 10 ns. Lifetime is preferably modulated by the nearby presence of another fluorophore or a quencher with which a resonant energy transfer occurs. Instruments for TRF simply delay the measurement of the emission until after the short-lived fluorescence has died out and the long-lived reporter fluorescence still persists. Fluorescence lifetime can be determined in two fundamental ways. The time domain technique uses very short pulses (picosecond) of excitation and then monitors the emission in real-time over the nanosecond lifetime. Fitting the decay curve to an exponential yields the lifetime. The frequency domain technique modulates the excitation at megahertz frequencies and then watches the emission intensity fluctuate in response. The phase delay and amplitude modulation can then be used to determine the lifetime. The frequency technique for fast and economical lifetime imaging is now available from Blueshift Biotechnologies Inc. As stated above, theses definitions apply to all of the described embodiments.

In one embodiment of the invention subject of the present invention is a method for simultaneous quantitative detection of multiple target nucleic acid sequences during amplification, wherein primers are labeled and the hybridized labeled amplicons are detected with a surface-specific detection system that detects those labels which are bound to the surface. Surface-specific means as defined above that labels which are in solution are essentially not detected.

In a preferred embodiment the signal of the label to be detected does not change in dependence of the binding state of said multiple nucleic acid sequences.

In an even more preferred embodiment the signal to be detected is a fluorescent signal. It is preferably an "always-on label" which are preferentially small organic fluorophores, but can also be a particulate label (either fluorescent or non-fluorescent), such as nano-phosphores, quantum dots.

For detection of multiple nucleic acids multiple labels may be used, in a preferred embodiment the same label is used for detection of each of said multiple target nucleic acid sequences.

In one embodiment of the method according to the present invention the capture probes are probes with a fluorescent label and a quencher in close proximity to one another due to the structure of the probes, and a signal may be detected in case an amplicon hybridizes to said capture probes.

Yet another embodiment of the present invention is a method for simultaneous quantitative detection of multiple target nucleic acid sequences, wherein the capture probes are immobilized on individually identifiable beads. In a preferred embodiment different beads have different capture probes.

It may be preferred that the beads are brought to the surface of the compartment and captured amplicons are measured by surface-specific detection. The beads may be brought to the surface e.g. by magnetic actuation.

In a preferred embodiment the fluorescence of a label is detected. As outlined above, a person skilled in the art knows further fluorescent and non-fluorescent labels.

If the capture probes are immobilized on beads, this allows for a quasi-homogeneous assay as the beads are freely dispersed in the reaction solution. Quasi-homogeneous assays allow for faster kinetics than non-homogeneous assays. The beads can have a diameter between 50 nm and 3 µm and are individually identifiable e.g. by color-coding, bar-coding or size. Multiple beads containing different capture probes may be present in the same reaction solution. Beads with captured amplicons on them can be brought to the surface by magnetic actuation (when (para)magnetic beads are used) or by dielectrophoresis (when non-magnetic beads are used). On the surface the beads can be identified and the captured amplicons can be detected by a surface-specific optical measurement.

Another method to distinguish different beads is based on the differences in resonance wavelength of the resonator modes propagating along the perimeter of the bead due to size differences. In this case the bead acts as a resonator that supports resonator modes propagating along the perimeter of the bead (for a spherical bead these resonator modes are so-called whispering gallery modes). The resonator is in-resonance for a wavelength where the phase shift after one roundtrip along the perimeter is a multiple of 2*pi. These resonator modes also have an evanescent field that extends into the environment (fluid) of the bead. A fluorophore in the near field region (typically <100 nm) of the bead can couple a fraction of its fluorescence to the resonator modes supported by the bead. The fraction coupled to the resonator mode is stronger for on-resonance wavelengths than for other wavelengths and this results in a modulation of the fluorescence spectrum with the resonance peaks corresponding to the resonator modes or whispering gallery modes. The typical diameter of the beads is larger than 1 µm up to 50 µm. Detection can be performed throughout the whole volume, because the fluorescence due to fluorophores that are not bound to the bead have the intrinsic spectrum of the fluorophore, and there is no need for a surface-specific optical measurement.

## Claims

1. A method for detecting a nucleic acid comprising:
providing a target nucleic acid to be detected,
a first step of amplifying the target nucleic acid,
a binding step in which amplified target nucleic acid binds to a surface-immobilized capture probe,
detecting said amplified target nucleic acid,
a step of measuring a melting curve of the amplified target nucleic acid bound to the capture probe before the second step of amplifying the target nucleic acid,
and a second step of amplifying the target nucleic acid.

2. A method according to claim 1, wherein the melting curve is analyzed for assessing the specificity with which the amplified target nucleic acid is bound to the capture probe.

3. A method according to claim 2, wherein the method further comprises a step of adjusting a parameter for controlling (a) subsequent step(s) of the method.

4. A method according to claims 1 to 3, wherein amplifying the target nucleic acid involves a temperature increase and wherein the temperature increase is used for measuring the melting curve.

5. A method according to claims 1 to 4, wherein amplifying the target nucleic acid involves PCR.

6. A method according to claims 1 to 5, wherein the amplification takes place in a container comprising a microarray.

7. A method according to claims 1 to 6, wherein the detection of said amplified nucleic acid sequences captured to said capture probes is performed with a surface-specific detection method detecting a signal in proximity to the surface.

8. A method according to claims 1 to 7, wherein the melting curves are measured and analyzed during more than one cycle of the amplification.

9. A method according to claims 1 to 8, wherein detection of amplified target nucleic acids is a quantitative detection.

10. A method according to claims 1 to 9, wherein multiple target nucleic sequences are simultaneously and real-time detected in a single container.

11. A method according to claims 1 to 10, wherein the detected signal is a fluorescence signal.

12. Device for performing a method according to claims 1 to 11 comprising:
a reaction compartment for the amplification of a target nucleic acid,
a hybridization surface comprising a surface-immobilized capture probe,
a detection system for detecting target nucleic acids captured to the surface-immobilized capture probes,
a melting curve measurement system comprising:
temperature varying and temperature control means,
a detection system for detecting target nucleic acids captured to the surface-immobilized capture probes.

13. A device according to claim 12, wherein the device comprises an algorithm that causes, when executed, said device to carry out the method steps according to the method of claims 1 to 11.

14. Use of a device according to claim 12 or 13 or use of a method according to claims 1 to 11 for a use selected from the group comprising clinical diagnostics, point-of-care diagnostics, bio-molecular diagnostics, gene or protein expression arrays, environmental sensors and food quality sensors.

15. Use of a device according to claim 12 or 13 or use of a method according to claims 1 to 11 for analyzing a sample for the presence of a target nucleic acid.
